# EUROPEAN PATENT SPECIFICATION

(11) **EP 0 553 155 B1**
(45) Date of publication and mention of the grant of the patent: **14.09.1994**
(21) Application number: 91917801.2
(22) Date of filing: 11.10.1991
(51) Int. Cl.: C07D 209/14, A61K 31/40

(54) **MELATONIN DERIVATIVE HAVING THERAPEUTIC ACTIVITY IN DERMATOLOGY**
MELATONINDERIVAT MIT THERAPEUTISCHER AKTIVITÄT, DAS IN DER DERMATOLOGIE ANWENDBAR IST
DERIVE DE MELATONINE PRESENTANT UNE ACTIVITE THERAPEUTIQUE EN DERMATOLOGIE

(30) Priority: 17.10.1990 IT 217689
(43) Date of publication of application: 04.08.1993
(73) Proprietor: PULITZER ITALIANA S.r.l., 00156 Roma (IT)
(72) Inventor: RAINOLDI, Angelo, I-00156 Rome (IT)
(74) Representative: Minoja, Fabrizio
(86) International application number: EP9101940
(87) International publication number: WO9206955

(56) References cited:
- WO-A-87/00432
- US-A- 4 746 674

## Description

The present invention relates to 5-methoxy-N-(10-undecenoyl)-tryptamine of formula 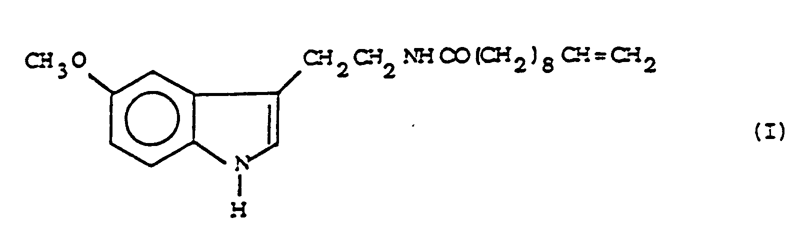
a process for the preparation thereof and pharmaceutical compositions containing it.

Melatonin is a hormon synthetised in epiphysis, retina and supposedly also in intestinal chromaffin cells. Melatonin biosynthesis follows a circadian rhythm and reaches its highest peak during night-hours.

The compound of the invention, which hereinafter will also be named PU 2049, is the condensation product from two pharmacologically active compounds: desacetyl melatonin, which is already well known to be active in the treatment of psoriasis (as described in WO 87/00432) and 10-undecenoic acid, which has a remarkable antimicrobic, particularly antimycotic, activity.

It has now been found that PU 2049 has a better bioavailability in the case of topic administration.

The pharmacological tests evidenced that PU 2049 is particularly effective in the treatment of psoriasis and further has a good antimycotic effect and an interesting antiseborrhoic activity.

PU 2049 activity in the treatment of psoriasis was evidenced during a double blind test, wherein 20 patients suffering from psoriasis at their scalp (7 patients), at their elbows (5 patients), at their knees (4 patients), at their palms and/or their soles. The subjects were divided into two groups and treated with daily topic applications of a 0.3% PU 2049 hydroalcoholic solution (1^{st} group) and melatonin (2^{nd} group), which was used as reference compound. In both groups, the treatment was continued for 20 days running.

PU 2049 proved to have a higher therapeutic activity in reducing skin desquamation and erythemapapulosae lesions than that of melatonin. In fact, all the patients treated with PU 2049 showed an improvement, both during and after the therapy, which was significantly more marked than the one seen in the control group.

The antimycotic activity of the compound of the invention was tested on agar culture dishes, according to the method described by Bennett G.A. et al. (Arzneim. Forsch./Drug Res. 40, 210; 1990). PU 2049 antimycotic activity was particularly evident against some dermatophytes strains, like epidermophytes, trichophytes, microspores. In these tests, melatonin was inactive.

Further, PU 2049 was tested against hamster flank glands. These glands are rich in sebaceous cells, therefore they are a suitable mean to evaluate drug antiseborroic activity, as described by Lutsky B.N. et al. (J. Invest. Dermatol., 64, 412; 1975). According to the present invention, a 0.3% PU 2049 hydroalcoholic solution or a melatonin hydroalcoholic solution were daily applied for 15 days running on flank skin of hamsters, weighing 90-100 g. A control group was treated with carrier only. At the end of the treatment, the glands of all the animals were withdrawn and weighed. The glands treated with PU 2049 showed a 45% weight decrease. Such an effect was significantly greater than the one obtained with melatonin.

It is therefore a further object of the present invention the use of PU 2049 as a therapeutic agent in formulations which can be prepared with conventional excipients and techniques, like those described in "Remington's Pharmaceutical Sciences Handbook" Mack Pub. Co., NY, USA.

The pharmaceutical compositions of the present invention contain from 1 to 500 mg PU 2049 and can be administered in one or more applications a day, according to symptom severity.

Examples of the above compositions are creams, ointments and oils, and every other formulation suitable for topic applications.

The compound of the invention can be prepared by reacting desacetyl melatonin or (5-methoxytryptamine) with 10-undecenoic acid or, more preferably, with a reactive derivative thereof (acid chloride, acid anhydride, imidazolide, and the like).

The use of the acid chloride comprises the presence of acid-binding agents (like pyridine, tertiary amines, alkali or alkaline-earth metal hydrogen carbonates) in inert solvents.

The following example further illustrates the invention.

### EXAMPLE

2.13 ml (0.01 mole) of 10-undecenoyl chloride were added to a solution (kept into darkness) containing 1.90 g (0.01 mole) of 5-methoxytryptamine, dissolved in 50 ml of a benzene-pyridine (7/3) mixture. The reaction mixture was allowed to stand for 1 hour, while stirring at reaction temperature.

The solvent was evaporated under reduced pressure and the residue was taken up with benzene.

The organic phase was washed with 10% HCl, 5% NaOH and water. The organic layer was dried over sodium sulphate, the solvent was evaporated under reduced pressure and the residue was triturated in hexane/isopropyl ether, obtaining 1.9 g of PU 2049.
**NMR (CDCl₃):**
δ (p.p.m.) 1.2-1.7 (multiplet), 12H (alkyl CH₂); 1.9-2.3 (complex signal), 4H (CH₂CH₂NH, CH₂CH=); 2.95 (triplet, J-7Hz), 2H (CH₂CO); 3.5-3.7 (double triplet, triplet after deuteration), 2H (CH₂NH); 3.87 (singlet), 3H (CH₃); 4.8-5.1 (multiplet), 2H (CH₂=); 5.5-5.9 (complex signal), 2H (CH=, NHCO); 6.7-7.3 (multiplet) 4H (aromatic); 8.3 (broad singlet disappears after deuteration), 1H (NH).
**IR (Nujol):**
frequency (cm⁻¹) 3400, 3300 (NH); 1640 (CO).

## Claims (Claims for the following Contracting State(s): AT, BE, CH, DE, DK, FR, GB, IT, LI, LU, NL, SE)

1. 5-Methoxy-N-(10-undecenoyl)-tryptamine of formula

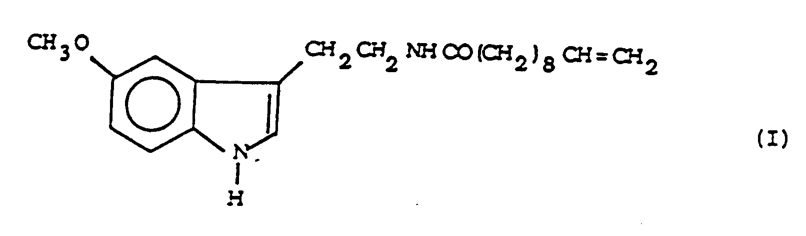

2. A process for the preparation of the compound of claim 1 characterized by reacting 5-methoxytryptamine with 10-undecenoic acid or with a reactive derivative thereof.

3. The compound of claim 1 as a therapeutic agent.

4. Pharmaceutical compositions containing the compound of claim 1 as the active ingredient, in admixture with suitable carriers.

5. The use of the Compound of claim 1 for the preparation of a medicament useful for the treatment of psoriasis and mycotic infective diseases.

## Claims (Claims for the following Contracting State(s): ES, GR)

1. A process for the preparation of 5-Methoxy-N-(10-undecenoyl)-tryptamine of formula 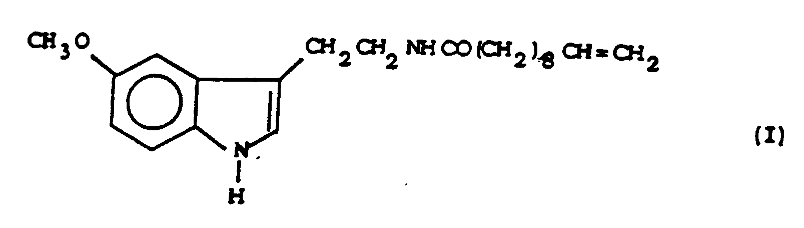 characterized by reacting 5-methoxytryptamine with 10-undecenoic acid or with a reactive derivative thereof.

## Patentansprüche (Patentansprüche für folgende(n) Vertragsstaat(en): AT, BE, CH, DE, DK, FR, GB, IT, LI, LU, NL, SE)

1. 5-Methoxy-N-(10-undecenoyl)tryptamin der Formel

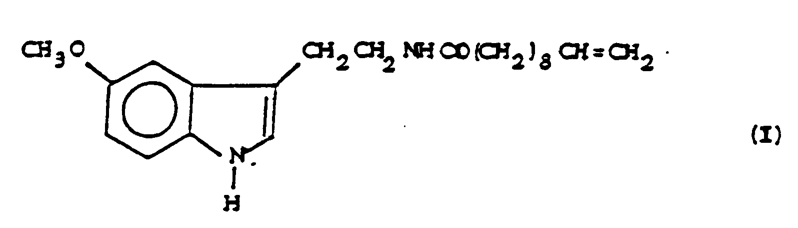

2. Verfahren zur Herstellung der Verbindung nach Anspruch 1, dadurch gekennzeichnet, daß man 5-Methoxytryptamin mit 10-Undecensäure oder deren reaktionsfähigem Derivat umsetzt.

3. Die Verbindung nach Anspruch 1 als therapeutisches Mittel.

4. Arzneimittel-Zusammensetzungen, enthaltend die Verbindung nach Anspruch 1 als Wirkstoff im Gemisch mit geeigneten Trägern.

5. Die Verwendung der Verbindung nach Anspruch 1 zur Herstellung eines Arzneimittels zur Behandlung von Psoriasis und infektiösen Pilzerkrankungen.

## Patentansprüche (Patentansprüche für folgende(n) Vertragsstaat(en): ES, GR)

1. Verfahren zur Herstellung von 5-Methoxy-N-(10-undecenoyl)-tryptamin der Formel 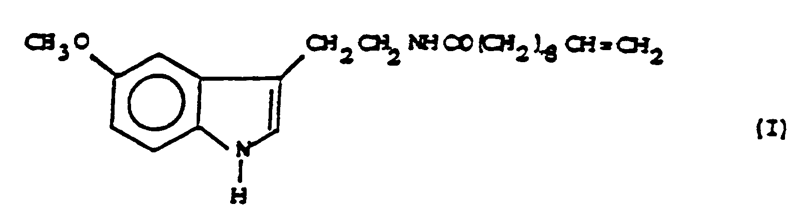 dadurch gekennzeichnet, daß man 5-Methoxytryptamin mit 10-Undecensäure oder deren reaktionsfähigem Devirat umsetzt.

## Revendications (Revendications pour l'(les) Etat(s) contractant(s) suivant(s): AT, BE, CH, DE, DK, FR, GB, IT, LI, LU, NL, SE)

1. 5-Méthoxy-N-(10-undécènoyl)-tryptamine de formule

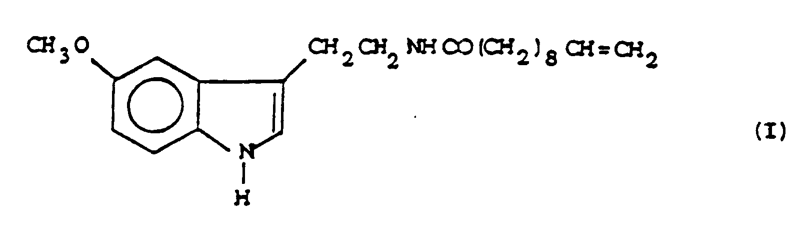

2. Procédé pour la préparation du composé de la revendication 1 caractérisé par la réaction de la 5-méthoxytryptamine avec l'acide 10-undécènoïque ou avec un dérivé réactif de celui-ci.

3. Composé de la revendication 1 comme agent thérapeutique.

4. Compositions pharmaceutiques contenant le composé de la revendication 1 comme ingrédient actif, en mélange avec des véhicules convenables.

5. Utilisation du composé de la revendication 1 pour la préparation d'un médicament utile pour le traitement du psoriasis et des maladies infectieuses mycosiques.

## Revendications (Revendications pour l'(les) Etat(s) contractant(s) suivant(s): ES, GR)

1. Un procédé de préparation de la 5-Méthoxy-N-(10-undécènoyl) -tryptamine de formule 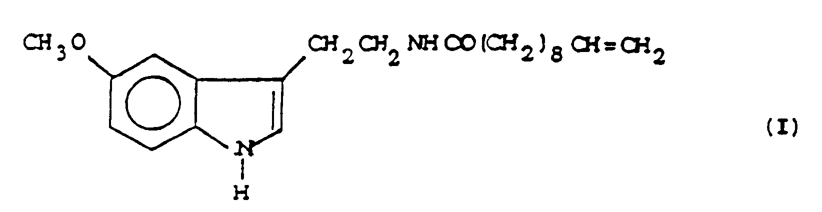 caractérisé par la réaction de la 5-méthoxytryptamine avec l'acide 10-undécènoïque ou avec un dérivé réactif de celui-ci.
